(19) Europäisches Patentamt
European Patent Office
Office européen des brevets

(11) **EP 2 891 480 B1**

(12) **EUROPEAN PATENT SPECIFICATION**

(45) Date of publication and mention
of the grant of the patent:
**22.07.2026 Bulletin 2026/30**

(21) Application number: **14150234.4**

(22) Date of filing: **06.01.2014**

(51) International Patent Classification (IPC):
*A61F 13/49* (2006.01)        *A61F 13/15* (2006.01)

(52) Cooperative Patent Classification (CPC):
**A61F 13/15699; A61F 13/49011;** A61F 2013/49022;
A61F 2013/49038

(54) **EXTENSIBLE LAMINATE**

Dehnbares Laminat

Stratifié extensible

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**08.07.2015 Bulletin 2015/28**

(73) Proprietor: **Ontex BV
9255 Buggenhout (BE)**

(72) Inventor: **Van Goethem, Jeroen
9255 Buggenhout (BE)**

(74) Representative: **Larangé, Françoise
Ontex BV
Korte Keppestraat 21
9320 Erembodegem (Aalst) (BE)**

(56) References cited:
WO-A1-2013/002691        US-A1- 2005 101 216
US-A1- 2008 033 387        US-A1- 2009 208 703

**Description**

**TECHNICAL FIELD**

The present invention relates to a laminate suitable for absorbent articles. More particularly, the invention relates to an elastic laminate composed of at least three layers, such as an elastic film sandwiched between two nonwoven webs, bonded to each other, to methods for manufacturing said elastic laminate, as well as to the use of an elastic laminate according to the invention in an absorbent article, particularly a pant diaper. Such an elastic laminate provides improved extensibility, softness, formability and thus comfort for the wearer. The present invention is of particular importance to the field of disposable hygiene products, in particular diapers, and more in particular pant-type diapers.

**BACKGROUND**

Absorbent articles having defined core regions and chassis regions are supposed to have a comfortable fit about the wearer. For pant articles like pant diapers, sanitary pants and incontinence pants it is also desirable that the articles are capable of being pulled up and down over the hips of the wearer to allow the wearer or caregiver to easily put on and remove the article when it has been soiled. It is thus known to make such absorbent pants with elasticized stretchable side panels and waist portion, usually comprising elastic members, such as elastic threads, contractably affixed between the backsheet and the topsheet.

It is further known to make portions of the chassis of absorbent articles of an elastic material, such as stretch-bonded laminates. Such laminates may include a layer of elastic film which have been sandwiched between outer layers of nonwoven webs. More particularly, the elastic film is typically bonded to the nonwoven web to form the stretchable elastic laminate.

Efforts have been done to improve the extensibility of the said absorbent article; however, there is still room for improvement with respect to comfort, fit and cloth-like feel of absorbent articles. Furthermore, the use of adhesives such as glue is still common in the manufacturing of absorbent articles which needs to be preferably prohibited due to environmental issues. Moreover, use of adhesives in high-volume production of absorbent articles, leads to increased costs and is therefore preferably limited or avoided all together.

U.S. Pat. No. US 8,052,665 B2 discloses an absorbent article comprising an elastic laminate. A problem with such an absorbent article is that the wearer expectations regarding extensibility are not fulfilled with such absorbent article because the elastic laminate is stiff. Furthermore, the layers from the elastic laminate are bonded with a pressure sensitive hot melt adhesive.

CA 2649926 discloses an elastically stretchable laminate in accordance with a method in which the elastically stretchable laminate is comprising at least three layers, the method including the steps of: a) producing a first laminate comprising a first non-elastic fibrous nonwoven web and an elastic film; b) activating the first laminate by incremental stretching in at least one direction to render the first laminate elastically stretchable; stretching the activated first laminate to 10-200% in at least one direction; and d) laminating the stretched first laminate to a second non-elastic nonwoven web. A problem with such an absorbent article is that pressure sensitive hot melt adhesive is also used during the processing of such elastic laminate because the second non-elastic nonwoven web is adhesively bonded to the activated first laminate.

Further prior art is disclosed in US2009/208703, WO2013/002691 and WO2009/133508.

The present invention aims to resolve at least some of the problems mentioned above. The present invention aims at providing an improved absorbent article of the above-described type with regard to wear comfort. This object is achieved in accordance with the invention by a disposable absorbent article comprising the features of the claims or as further specified herein. Furthermore, the present invention aims at providing an elastic laminate with improved extensibility and softness, and whereby the use of adhesive can be kept low or can be avoided, leading to reduced production costs and to a more environment-friendly product than prior art laminates.

**SUMMARY OF THE INVENTION**

The present invention concerns an absorbent article, preferably a disposable hygiene article, such as pant diapers, sanitary pants, incontinence pants, and the like, but also diapers, baby diapers, incontinence diapers, etc. Such an absorbent article may comprise a chassis comprising a core region holding an absorbent core (11), said chassis preferably further comprising a front panel, a back panel and an elastic laminate (10), each of the front and back panels having a waist edge, a crotch edge and a pair of side edges, wherein the front and back panels being joined to each other along two opposite side edges to define a waist-opening and a pair of leg-openings, at least one of the front and back panels comprises an elastic laminate.

In a first aspect, the present invention specifically concerns an elastic laminate as defined in claim 1.

According to the Kawabata KES-F system, the said elastic laminate preferably comprises an extensibility which is higher than 40 %, preferably higher than 45 %, more preferably higher than 50 %, yet more preferably higher than 55%.

In a preferred embodiment, said elastic laminate comprises an average extensibility which is higher than 22 %, preferably higher than 25 %, more preferably higher than 30 %, yet more preferably higher than 35%, still more preferably higher than 40%, even more preferably higher than 45%, yet even more preferably higher than 50%, still even more preferably higher than 55%, whereby the average is taken by testing the extensibility of the laminates in ten articles of the same type.

The first nonwoven web and the second nonwoven web are laminated to the elastic film in a discontinuous bonding region, preferably according to a discontinuous bonding pattern which can be pre-defined, preferably said pattern being regular. The elastic film is extended at least locally during lamination. Both previous features, and in particular the combination, lead to improved extensibility of more than 40%, up to 60% or even more. Furthermore, they also lead to a bulky laminate, which in turn leads to increased softness of the laminate.

The first and second nonwoven webs (4 and 6) are bonded, preferably mechanically, thermally and/or thermo-mechanically bonded to the elastic film (5). For example, ultrasonic welding can be used for bonding the first and/or second nonwoven webs (4 and 6) to the elastic film (5). This method bonds the elastic laminate with heat generated from ultrasonic oscillation and laminates each layer to each other. Upon cooling and shaping, the elastic laminate's layers are joined together. Preferably, the elastic laminate contains less than 1 $g/m^2$ of adhesive, preferably less than 0,5 $g/m^2$, more preferably about 0 $g/m^2$.

The elastic laminate comprises a softness which is higher than 13, preferably higher than 16, more preferably higher than 20, still more preferably higher than 25, yet more preferably higher than 31 and/or a formability which is higher than 20, preferably higher than 22, more preferably higher than 23, yet more preferably higher than 24 according to Kawabata and/or a TS750 peak of at least 200 dB $V^2_{rms}$ if the laminate comprises a TS7 peak of at most 25 dB $V^2_{rms}$, as measured according to the tissue softness analyser. Said softness could be measured, or preferably, according to the tissue softness analyser as specified further below in this document.

[first and/or preferably the second]

The nonwoven comprise:

- an elongation in a machine direction of at least 60%;
- a stiffness in a machine direction of at most 35 mN;
- a stiffness in a cross direction of at most 20 mN, and/or
- a kinetic coefficient of friction (COF kin) of at most 0,30.

Preferably, herein,

- the elongation in the machine direction is measured according to the WSP test 110.4;
- the stiffness in the machine and in the cross direction are measured according to the WSP test 090.3, and/or
- the COF kin is measured according to ASTM test D1894.

WSP tests refer to Worldwide Strategic Partners (WSP) test methods. These test methods are the harmonized results of INDA's (Association of the Nonwoven Fabrics Industry) and EDANA's (European Disposables And Nonwovens Association) efforts to develop worldwide test methods for the worldwide nonwovens industry:

- Test WSP110.4 refers to a Standard Test Method for Breaking Force and Elongation of Nonwoven Materials (Strip Method);
- Test WSP090.3 refers to a Standard Test Method for Handle-O-Meter Stiffness of Nonwoven Fabrics.

More information about the WSP tests can be found in the tables below.

| TITLE | Breaking Force Strip Test |
|---|---|
| METHOD NUMBER | WSP 110.4 |
| IST/ERT REFERENCE | IST 110.5 (02)/ERT 20.2 (89) |
| ISO | |
| PAGE | 11.17 |

(continued)

| TITLE | Breaking Force Strip Test |
|---|---|
| DESCRIPTION | **STANDARD TEST: WSP 110.4 (05)**<br>**Standard Test Method for Breaking Force and Elongation of Nonwoven Materials (Strip Method)**<br>***This (04) version includes both INDA IST 110.4 (02) (Option A) and EDANA ERT 20.2-89 (Option B)***<br>This test method covers cut strip test procedures for determining the breaking force and elongation of most nonwoven materials. Instructions are included for wet testing. This test method describes two procedures Option A - IST 110.4 -02 and Option B - ERT 20.2 - 89 for carrying out nonwoven material tensile tests. These two procedures use two types of specimens which are listed below and three alternative types of testing machines are also listed below. CRE is the instrument of choice.<br>The number in parentheses indicates the year of the last revision |

| TITLE | Handle-O-Meter |
|---|---|
| METHOD NUMBER | WSP 90.3 |
| IST/ERT REFERENCE | Modified IST 90.3 (01) |
| ISO | |
| PAGE | 9.15 |
| DESCRIPTION | **STANDARD TEST: WSP 90.3. (05)**<br>**Standard Test Method for Handle-O-Meter Stiffness of Nonwoven Fabrics**<br>This test method covers the evaluation of the stiffness or "hand" of nonwovens. The quality of "hand" is considered to be the combination of resistance due to the surface friction and the flexural rigidity of a sheet material (see ANNEX A).<br>All of the instruments operate on the principle of deforming the nonwoven through a restricted opening. It is a flexibility test in which the force required to deform a loop of nonwoven is measured.<br>The number in parentheses indicates the year of the last revision |

The ASTM D1894 test refers to a Standard Test Method for Static and Kinetic Coefficients of Friction of Plastic Film and Sheeting. This test method covers determination of the coefficients of starting and sliding friction of plastic film and sheeting when sliding over itself or other substances at specified test conditions. The procedure permits the use of a stationary sled with a moving plane, or a moving sled with a stationary plane. Both procedures yield the same coefficients of friction values for a given sample. It has ICS Number Code 83.140.10 (Films and sheets) and the standard is disclosed in DOI: 10.1520/D1894-11E01.

In a second aspect, the present invention provides an absorbent article comprising an elastic laminate according to an embodiment of the invention. Preferably, said article comprises a nonwoven front sheet and a nonwoven back sheet, and an elastic film laminated therebetween, thereby forming said elastic laminate, more preferably whereby said laminate is at least partly disposed in a front section and/or a back section of said absorbent article.

In a third aspect, the invention also provides a process for manufacturing an elastic laminate according to an embodiment of the invention, as defined in claim 8.

Preferably, the elastic film of the present invention is extended in at least one direction, e.g. by 400%, during lamination to the first and/or preferably also the second nonwoven web. In a preferred embodiment, the elastic laminate used is used in an absorbent article and is extensible in at least a transverse direction of the absorbent article.

In a further aspect, the invention provides use of an elastic laminate according to an embodiment of the invention for manufacturing an absorbent article.

The elastic laminate provided by the invention can be particularly advantageous for use in an absorbent article such as a pant diaper.

Preferred embodiments are as specified in the dependent claims and as further below.

## BRIEF DESCRIPTION OF THE DRAWINGS

The invention will be further described in detail with reference to the exemplary embodiments represented by the accompanying figures, in which

**FIG. 1** shows a general schematic cross-sectional view of an elastic laminate comprising a nonwoven web (1) and an elastic film (2).

**FIG. 2** shows a general schematic cross-sectional view of an elastic laminate comprising an elastic film (5) sandwiched between two nonwoven webs (4 and 6) according to the present invention.

**FIGS. 3 and 4** show a perspective view and detail of a pant diaper comprising an elastic laminate (10) according to the present invention.

**FIG. 5** illustrates the measurement of elongation according to KES-FB01.

**FIG. 6** illustrates the measurement of compressional properties according to KES-FB03.

**FIGS. 7a-c** illustrates the measurement of surface and geometrical roughness according to KES-FB04.

**FIG. 8** illustrates the tensile energy which represents the force needed to elongate the material and which is a relative value for the strength of the material.

**FIGS. 9a-b** illustrate the principle and test conditions for the TSA.

**FIGS. 10a-b and 11** illustrate the results of the TSA on samples of the laminate according to the present invention and comparison with Market Standard and competitive laminate.

## DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

As used herein, the following terms have the following meanings:

"Absorbent article" refers to devices that absorb and contain liquid, and more specifically, refers to devices that are placed against or in proximity to the body of the wearer to absorb and contain the various exudates discharged from the body.

Absorbent articles include but are not limited to diapers, adult incontinence briefs, training pants, diaper holders and liners, sanitary napkins and the like. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn.

The absorbent article of the present invention is preferably a pant diaper comprising a front section and a back section, an absorbent material and an elastic laminate at least partly disposed in the front and/or back sections. The elastic laminate comprising a three-layer structure comprising an elastic film laminated between two nonwoven webs, preferably a front sheet and/or a back sheet of the absorbent article, the webs bonded to the elastic film, preferably by mechanical, thermal and/or thermo-mechanical bonding, such as by ultrasonic welding.

"Absorbent medium" is the absorbent structure disposed between the top sheet and the back sheet of the absorbent article in at least the crotch region of the absorbent article. The absorbent material can be of any conventional kind. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material. Superabsorbent polymers are water-swellable, water-insoluble organic or inorganic materials capable of absorbing at least about 20 times their own weight of an aqueous solution containing 0.9 weight percent of sodium chloride. Organic materials suitable for use as superabsorbent materials can include natural materials such as polysaccharides, polypeptides and the like, as well as synthetic materials such as synthetic hydrogel polymers. Such hydrogel polymers include, for example, alkali metal salts of polyacrylic acids, polyacrylamides, polyvinyl alcohol, polyacrylates, polyvinyl pyridines, and the like. Other suitable polymers include hydrolyzed acrylonitrile grafted starch, acrylic acid grafted starch, and isobutylene maleic anhydride copolymers and mixtures thereof. The hydrogel polymers are preferably lightly cross-linked to render the material substantially water insoluble. Preferred superabsorbent materials are further surface cross-linked so that the outer surface or shell of the superabsorbent particle, fibre, flake, sphere, etc. possesses a higher crosslink density than the inner portion of the superabsorbent. The superabsorbent materials may be in any form which is suitable for use in absorbent composites including particles, fibres, flakes, spheres, and the like.

"Chassis" refers to a foundational constituent of an absorbent article upon which the remainder of the structure of the article is built up or overlaid, e.g., in a diaper, the structural elements that give the diaper the form of briefs or pants when configured for wearing, such as a backsheet, a topsheet, or a combination of a topsheet and a backsheet.

"Diaper" refers to an absorbent article generally worn by infants and incontinent persons about the lower torso.

"Discontinuous bonding pattern" as used herein refers to a pattern of bonding areas, in particular bonding areas between layers comprised in the laminate of the present invention, whereby at least in at least one region the layers are not bonded. A discontinuous bonding pattern may comprise a connected bonding area or multiple disconnected bonding areas. A discontinuous bonding pattern further may comprise a connected bonding area comprising a number of holes, where the layers are not bonded, preferably according to a regular pattern, or it may comprise discrete disconnected bonding areas, e.g. a point bonded pattern which comprises a plurality of separate bonding points surrounded by unbonded areas or a line-bonded pattern which comprises a plurality of separate bonding lines alternated by unbonded areas, preferably according to a regular pattern.

"Disposable" is used herein to describe articles that are generally not intended to be laundered or otherwise restored or reused (i. e. , they are intended to be discarded after a single use and, preferably, to be recycled, composted or otherwise disposed of in an environmentally compatible manner).

"Laminate" refers to elements being attached together in a layered arrangement.

"Nonwoven" refers to a manufactured sheet, web, or batt directionally or randomly oriented fibres bonded by friction and/or cohesion and/or adhesion, excluding paper and products that are woven, knitted, tufted stitchbonded incorporating binding yarns or filaments, or felted by wet-milling, whether or not additionally needled. The fibres may be of natural or man-made origin. They may also be staple or continuous filaments or be formed in situ.

"Stretchable" or "extensible" refer herein to a material that can be stretched, without substantial breaking, by at least 10%, more preferably at least 20%, yet more preferably at least 30%, still more preferably at least 40%, even more preferably at least 50% of its relaxed, initial length in at least one direction. The term can include elastic materials, as well as nonwovens that can be inherently extensible, but not necessarily in an elastic manner. Such nonwovens can be made to behave in an elastic manner by bonding them to elastic films.

"Stretch" or "extension" refer herein to the additional length that a material reaches when a certain force is applied, leading to an extended state of the material beyond the initial length without breaking the material. An extension of f.e. 50% means that the material with an initial length of 100mm has reached a length of 150mm.

"Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

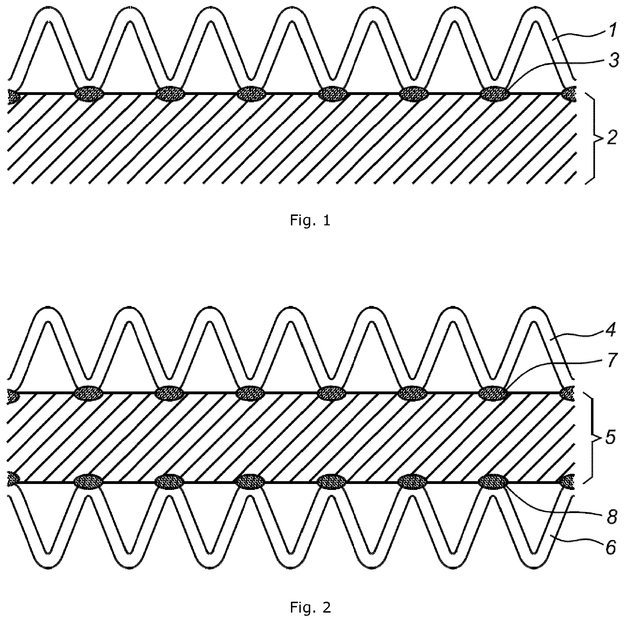

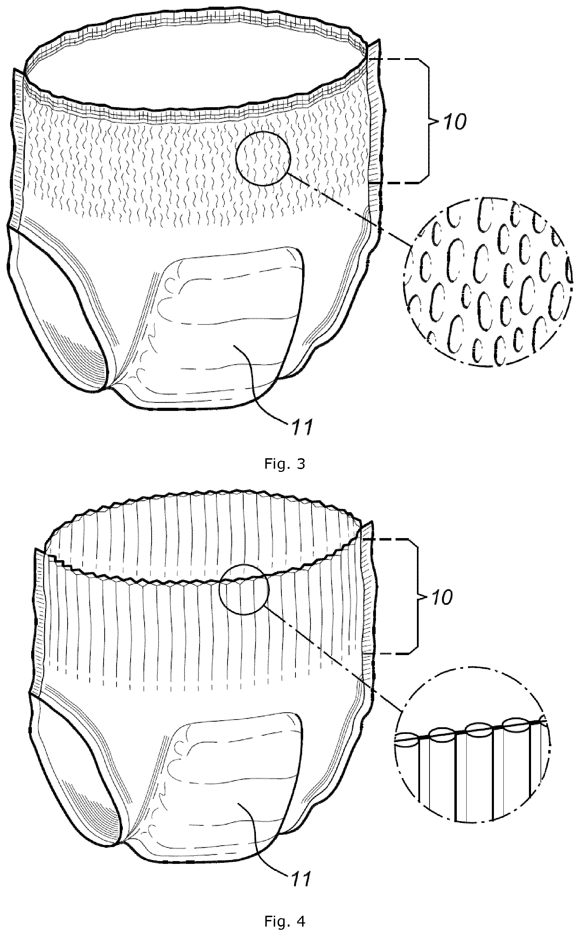

**FIG. 1** shows a general schematic cross-sectional view of an elastic laminate comprising a nonwoven web (1) and an elastic film (2); **FIG. 2** shows a general schematic cross-sectional view of an elastic laminate comprising an elastic film (5) sandwiched between two nonwoven webs (4 and 6) according to the present invention. **FIG.** 3 shows a perspective view of a pant diaper comprising an elastic laminate (10) according to the present invention.

The inventors have found a way to provide an improved elastic laminate and process for making such a laminate.

In particular, the present invention provides in a first aspect an elastic laminate for absorbent articles comprising a first nonwoven web (4), a second nonwoven web (6) and an elastic film (5) laminated between the first (4) and the second nonwoven (6) webs **(Fig. 2).**

Preferably, said elastic laminate comprises an extensibility according to Kawabata which is higher than 40 %, preferably higher than 50 %, more preferably higher than 60 %, yet more preferably higher than 80%. One of the advantageous feature of the absorbent article is that the elastic laminate is stretchable thereby improving the fit and comfort level of the wearer. Furthermore, the stretchable elastic laminate gives a desired level of strength to the waist band which could not be experienced in prior art absorbent article. Herein, the level of strength refers to the tensile energy (WT) according to Kawabata, which preferably is at least 12 gF/cm.

The elastic laminate comprises a softness which is higher than 13, preferably higher than 16, more preferably 20, even more preferably higher than 25, yet more preferably higher than 31 according to Kawabata and/or a TS750 peak of at least 200 dB $V^2_{rms}$ if the laminate comprises a TS7 peak of at most 25 dB $V^2_{rms}$, as measured according to the tissue softness analyser. The softness of the elastic laminate presents the advantage that it provides a feel comfort for the wearer. Thereby, preferably the elastic laminate had a soft outer and inner surfaces covering the lower torso of the wearer, which gives an improved softness and wearing comfort which could not be experienced in prior art absorbent articles. Indeed, the inner and outer surfaces are preferably formed of a soft material that will not irritate the skin of the wearer and/or the caregiver. Said softness could be measured according to Kawabata, or preferably, according to the tissue softness analyser as specified further below in this document.

In a preferred embodiment, the elastic laminate comprises a formability which is higher than 20, preferably higher than 22, more preferably higher than 23, yet more preferably higher than 24. One of the advantageous feature of the absorbent article is that the elastic laminate is stretchable with a satisfactory formability thereby increasing the fit and comfort level of the wearer.

A further advantage of the present invention, is that the said elastic laminate is formed by joining it by means of ultrasonic welding; The elastic film (5) is joined to the nonwoven webs (4 and 6) by melting them with heat generated from ultrasonic oscillation whereby at least the elastic film is stretched, e.g. by being put under tension, when being laminated resulting in an elastic laminate. The present invention is not limited to ultrasonic welding for bonding the elastic film to the nonwoven webs but to bonding in general, such as chemical, mechanical, thermal, thermo-mechanical bonding and/or bonding by adhesives, such as hot-melt adhesives, but preferably mechanical, thermal and/or thermo-mechanical bonding.

The [bonding] is done according to a discontinuous bonding pattern, preferably said pattern being regular. Such a pattern, preferably in combination with the elastic film being extended at least locally during welding, leads to a bulky laminate, which in turn leads to increased softness of the laminate.

The elastic laminate may comprise good tensile strength and low permanent deformation properties. The present invention is directed to a stretchable laminate, the laminate preferably being stretchable in at least one direction, that comprises at least one or two nonwoven webs (4 and 6), and an elastic film (5) attached to one nonwoven web or sandwiched between the two nonwoven webs (4 and 6), wherein said elastic film (5) is stretchable in at least the same one direction as the laminate. If the laminate is used in an absorbent article, the laminate is preferably stretchable in at least a transverse direction of the absorbent article. Furthermore, said stretchable laminate comprises a softness which is higher than 13, preferably higher than 16, more preferably higher than 20, still more preferably higher than 25, yet more preferably higher than 31 according to Kawabata and/or a TS750 peak of at least 200 dB $V^2_{rms}$ if the laminate comprises a TS7 peak of at most 25 dB $V^2_{rms}$, as measured according to the tissue softness analyser. Said softness could be measured according to Kawabata, or preferably, according to the tissue softness analyser as specified further below in this document.

The elastic laminate is composed of nonwoven webs that are bonded, preferably mechanically, thermally and/or thermo-mechanically bonded, more preferably ultrasonic welded to the elastic film.

The elastic laminate contains less than 1 $g/m^2$ of adhesive, preferably less than 0.5 $g/m^2$, e.g. 0.4, 0.3, 0.2, 0.1 $g/m^2$, more preferably about 0 $g/m^2$. Frequently, one or more elastomeric components of a disposable absorbent article are adhesively bonded together. For example, adhesives have been used to bond individual layers of the absorbent article together. In many cases, the bonding together of components forms a laminated structure in which adhesive is sandwiched between materials (such as layers of polymer film and/or layers of woven or non-woven fabrics) that make up the components being bonded together. In many instances, a hot-melt adhesive, i.e. a polymeric formulation is used in making a laminated structure. While such formulations generally work, they can be costly and their performance properties can be improved. Furthermore, the environmental impact is important thereby preferably limiting the use of such adhesives.

One of the advantageous feature of the absorbent article is that the elastic laminate of the present invention can be made comprising less than 1 $g/m^2$ of adhesive, preferably less than 0.5, 0.4, 0.3, 0.2, 0.1 $g/m^2$, more preferably about 0 $g/m^2$.

In a second aspect, the invention provides an absorbent article comprising an elastic laminate according to an embodiment of the invention.

In a third aspect, the invention provides a process for manufacturing an elastic laminate comprising the steps of providing an elastic film (5) wherein the elastic film is bonded, preferably mechanically, thermally and/or thermo-mechanically bonded to the nonwoven webs (4 and 6) thereby providing the elastic laminate.

In a preferred embodiment, the invention provides a process wherein the elastic film (5) and the nonwoven webs (4 and 6) are preferably bonded by ultrasonic welding, wherein said elastic laminate has a softness which is higher than 16.

In a preferred embodiment, the invention provides a process wherein the elastic laminate has an extensibilty in the transverse direction of the absorbent article of at least 40%, more preferably higher than 50 %, yet more preferably higher than 60%, when measured according to the extension test specified in the examples section.

The invention provides a process for manufacturing an elastic laminate as defined in claim 8.

The elastic film is stretched during lamination, preferably by at least 10%, more preferably at least 20%, yet more preferably at least 50%, still more preferably at least 100%, even more preferably at least 200%, yet even more preferably at least 300%, still even more preferably at least 350%, yet still more preferably by at least 375%. In a preferred embodiment, the elastic film is stretched during lamination for less than 550%, more preferably less than 500%, still more preferably less than 450%. In a particularly preferred embodiment, the elastic film is stretched by about 400%.

Note that stretching the elastic film more during production allows to obtain laminates comprising an extensibility of more than 40% and even up to 60% or more, while keeping the production costs low, or, a low-cost elastic film can be used to make a laminate comprising an extensibility of more than 40% and even up to 60% or more by appropriately extending the film during lamination.

Said laminating is done by bonding, more preferably by mechanical, thermal and/or thermo-mechanical bonding, still more preferably by ultrasonic welding, and this according to a discontinuous bonding pattern, preferably said pattern being regular. Such a pattern, preferably in combination with the elastic film being extended at least locally during bonding, leads to improved extensibility of the laminate, and also leads to a bulky laminate, which in turn leads to increased softness of the laminate. Improved extensibility and improved softness both increase comfortability for the wearer of the absorbent article separately, but particularly if combined.

In a further aspect, the invention provides use of an elastic laminate for manufacturing an absorbent article.

The present invention also provides an elastic laminate comprising a first nonwoven web (4), a second nonwoven web (6) and an elastic film (5) laminated between the first (4) and the second nonwoven (6) webs, and preferably further comprising any or any combination of the following:

1. An extension (EMT) measured according to Kawabata which is higher than 50%, i.e. EMT > 50%.
2. a material strength which remains equal during extension. This means that the force needed to extend our laminate remains stable during extension. This is measured by the Tensile linearity according to Kawabata (LT), which is higher than 0.9 and preferably about 1.0, i.e. LT > 0,9 and preferably LT about 1.0.
3. a Tensile energy (WT) according to Kawabata whereby $WT \geq 12$ gF/cm, which results in a unique elastic behavior of our laminate resulting in an improved fit and wearing comfort (together with the soft nonwovens we selected). Note that the tensile energy refers to the level of strength of the laminate, and/or
4. plies created by using ultrasonic welding, which result in a bulky material. Preferably soft nonwovens are used which together result in a material which is very soft and bulky, quantified by the compression energy (WC) measured according to Kawabata. This parameters quantifies the energy needed to compress the material to a certain extend. In a preferred embodiment, $WC \leq 0,65$ gF/cm
5. the laminate may comprise a soft 'wave' pattern, created preferably in combination with the soft nonwovens, resulting in an unique bulk softness, quantified according to Kawabata by the roughness (SMD). In a preferred embodiment $SMD \leq 5$ μ. Said softness could also be quantified with the tissue softness analyser.
6. the laminate comprises a softness: S = EMT/ SMD + 1/WC, whereby S > 13. Said softness could be measured according to Kawabata, or preferably, according to the tissue softness analyser as specified in this document.
7. the laminate may comprise a formability according to Kawabata: $F = WT + (EMT)^{1/2}$, whereby F > 20
8. the laminate may comprise a delamination force in the machine direction MD which is needed to separate the outer layer from the elastic film, which is between 1 and 3 N
9. the laminate may comprise a delamination force in the cross direction CD, which is needed to separate the outer layer from the elastic film, which is between 1 and 3 N, and/or

Hereby, the ultra-sonic technology in combination with the soft nonwovens result in an bulky soft waist material unique in e.g. a pull up diaper.

Although the present invention has been described with reference to preferred embodiments thereof, many modifications and alternations may be made by a person having ordinary skill in the art without departing from the scope of this invention which is defined by the appended claims.

The invention is described in greater detail in the examples below, which are given as non-limiting illustrations. In these examples, the temperature is room temperature or is expressed in degrees Celsius, and the pressure is atmospheric pressure. The water and all the reagents used are of injectable grade.

Moreover, all the examples form an integral part of the invention, as does any characteristic of the description including the examples, which appears to be novel with respect to any prior art, in the form of a general characteristic rather than of a particular characteristic of the example.

**Evaluation of the elastic laminate and examples**

### 1. Objective measurement system: KES-F

The Kawabata Evaluation System for Fabrics. Kawabata designed the Kawabata Evaluation System for Fabrics ("KES-F") system in 1972 to address a need for instrumentation that would enable fabric parameters to be objectively measured quickly, accurately, and reproducibly (see, e.g. Kawabata, S.; Niwa, M.; & Wang, F. "Objective Hand Measurement of Non Woven Fabrics," Textile Research J p. 597 (1994); Bishop, D. P. "Fabrics: Sensory and Mechanical Properties," Textile Progress Vol. 26, No. 3 (1995), and Matsuo, T.; Nasu, N. & Saito, M. "Study on the Hand. Part II. The Method of Measuring Hand," J. Text. Mach. Soc. Jap 17.3 p. 92, (1971)).

The KES system of instruments measures properties of textile fabrics and predicts the aesthetic qualities perceived by human touch. The KES system consists of four instruments: KES-FB**1** tensile and shear tester, KES-FB**2** bending tester, KES-FB**3** compression tester, and KES-FB**4** surface-friction and geometrical-roughness tester. The parameters measured by the basic KES-F (B) instruments are the following: bending properties, surface properties (friction and roughness), compression properties, shearing properties, and tensile properties. Each of these categories is comprised of a group of related mechanical properties that can be separately measured (see table below).

| Test | Parameter | description | Unit |
|---|---|---|---|
| Tensile | EMT | extension | % |
| | LT | linearity | - |
| | WT | Tensile energy | $gF\ cm/cm^2$ |
| | RT | Resilence | % |
| Bending | B | bending resistance | $gF\ cm^2/cm$ |
| | 2HB | bending hysteresis | gFcm/cm |
| Shear | G | Shear resistance | gF/cm grado |
| | 2HG | shear hysteresis | gF/cm |
| Compression | LC | linearity | - |
| | WC | compression | $gF\ cm/cm^2$ |
| | RC | Resilence | % |
| Surface | MIU | COF | - |
| | MMD | mean deviation | - |
| | SMD | roughness | $\mu$ |
| Thickness | T0 | thickness | mm |
| Weight | W | weigth | $mgF/cm^2$ |

As mentioned, the Kawabata KES-FB test is a Japanese quality judgment system for used for textile materials and is disclosed in "The Standardization and Analysis of Hand Evaluation (2nd Edition), Sueo Kawabata, July 1980, The Hand Evaluation and Standardization Committee, The Textile Machinery Society of Japan". The test used in this invention uses three of the Kawabata testing machines:

- KES-FB1 for measuring the Tensile strain, EMT (%), the Tensile energy WT ($gfcm/cm^2$) and Linearity of the load extension curve LT (-)
- KES-FB3 for measuring the Compression energy WC ($gf\ cm/cm^2$)
- KES-FB4 for measuring the Geometrical roughness ($\mu$)

For the present invention, the sample of interest comprises nonwovens whereby the total hand value (T.H.V.) was calculated according to the KN-304 Summer/winter equations.

Herein,

- **EMT = Tensile Strain - measured with KES-FB01**
- **LT** = **linearity of the load-extension curve - measured with KES-FB01**

- **WT = Tensile energy - KES -FB01**

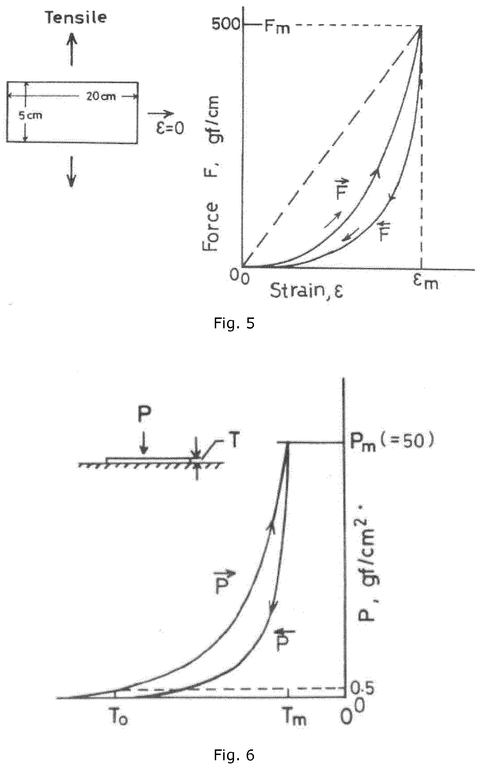

Measurements were performed in both direction (MD and CD) with the following settings (and with reference to **FIG.** 5):

Sample area: 20x20 cm
Max load: F=500 gf/cm
Tensile speed: 0.2mm/sec
Effective sample: 20 cm width and 5 cm in length
Elongation in length direction

$$LT = \int_0^{\varepsilon_m} \vec{F} \, d\varepsilon \Big/ (0.5 \, F_m \, \varepsilon_m)$$

$$WT = \int_0^{\varepsilon_m} \vec{F} \, d\varepsilon$$

**Compression energy** - **WC [gFcm/cm$^2$] KES-FB3**

The compressional properties was measured between two plates with increasing the pressure while continuously monitoring the sample thickness up to a max. pressure of 50gf/cm$^2$. See also **FIG. 6.**

Sample area: 20x20 cm
Compression rate: 20$\mu$m/sec
P0= 0.5 gf/cm$^2$
Maximum pressure: Pmax = 50gf/cm$^2$

$$WC = \int_{T_m}^{T_o} \vec{P} \, dT$$

**Geometrical roughness** - **SMD [$\mu$]** - **KES-FB4**

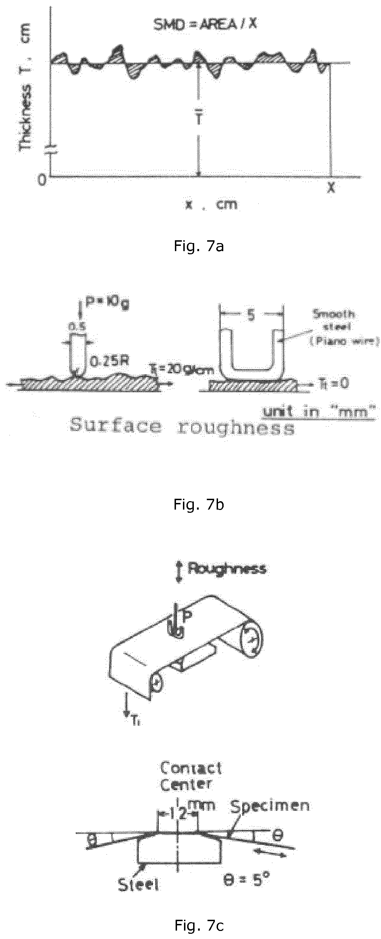

The mean deviation of the surface contour is measured, with reference to **FIGS. 7a-c.**

$$SMD = \frac{1}{X} \int_0^X |\, T - \bar{\bar{T}} \,| \, dx.$$

x = displacement of the sensor on the specimen
X = maximum of x, equal to 2 cm
Rate of sensor displacement on specimen surface = 0.1 cm/sec

2. **Basic properties affecting sensorial comfort**

2.1. <u>Tensile properties</u>

Tensile strength is a basic indicator of relative strength and is fundamental for materials that function primarily in tension. The extension (EMT) of a material is therefore an important factor for the comfort and softness of the material. Related to the extension capacity of a material is the energy needed for this extension, the tensile energy (WT).

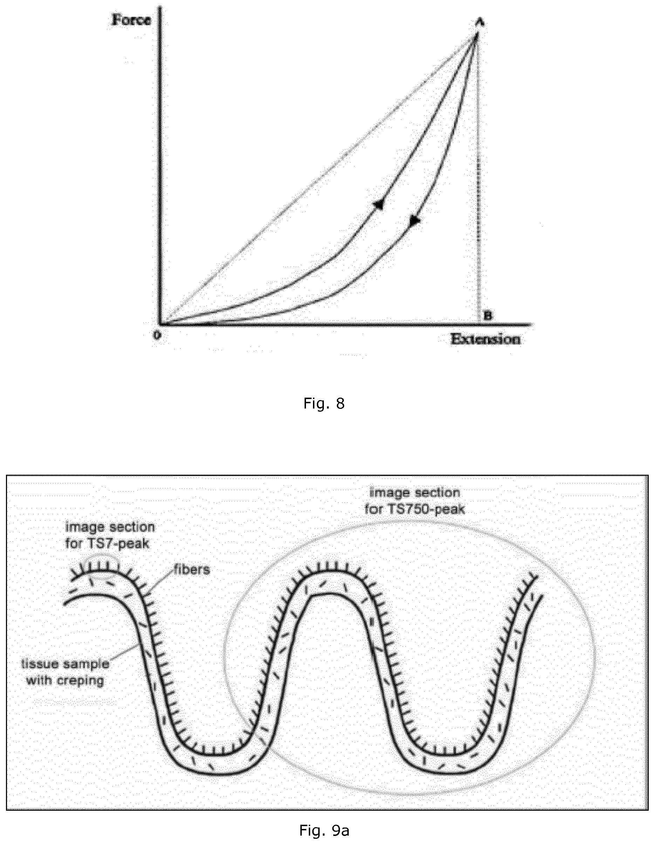

The tensile energy represents the force needed to elongate the material and is a relative value for the strength of the material (see **FIG. 8).** The linearity (LT) indicates how the material elongates:

LT < 1: the initial elongation requires less force than at the end.

LT = 1: the force needed to elongate the material is equally spread during extension.

LT > 1: the initial elongation requires more force than at the end.

In the present invention, the laminate is a three layer structure with two soft nonwovens and an elastic film, ultrasonic laminated to each other. The elastic film is put under a specific tension and gives flexibility properties to the laminate.

An important tensile property namely the extension (EMT) was determined for the elastic laminate according to the present invention compared to a market standard and a lycra waist, besides the tensile linearity, tensile energy, compression energy and roughness (See Table below, wherein "Par." = "Parameter" and "Avg." = "Average").

| Market standard - underwear like pull up | | | | | | | |
|---|---|---|---|---|---|---|---|
| **Test** | **Par.** | **description** | **Unit** | **Avg.** | **Stdev** | **Min** | **Max** |
| Tensile | EMT | extension | % | 21,45 | 9,80 | 16,28 | 38,88 |
| | LT | linearity | - | 0,84 | 0,11 | 0,66 | 0,91 |
| | WT | Tensile energy | $gF{*}cm/cm^2$ | 4,75 | 2,48 | 2,78 | 9,05 |
| Compression | WC | compression energy | $gF\,cm/cm^2$ | 0,88 | 0,49 | 0,21 | 1,47 |
| Surface | SMD | roughness | $\mu$ | 6,52 | 0,83 | 5,30 | 7,35 |
| Classic pull up - lycra waist (Ref. 3) | | | | | | | |
| **Test** | **Par.** | **description** | **Unit** | **Avg.** | **Stdev** | **Min** | **Max** |
| Tensile | EMT | extension | % | 79,04 | 6,47 | 70,70 | 87,75 |
| | LT | linearity | - | 0,81 | 0,04 | 0,75 | 0,85 |
| | WT | Tensile energy | $gF{*}cm/cm^2$ | 15,30 | 1,53 | 13,50 | 17,65 |
| Compression | WC | compression energy | $gF\,cm/cm^2$ | 1,29 | 0,62 | 0,25 | 1,75 |
| Surface | SMD | roughness | $\mu$ | 3,05 | 0,08 | 2,96 | 3,12 |
| Ontex laminate - underwear like pull up (Ref. 4) | | | | | | | |
| **Test** | **Par.** | **description** | **Unit** | **Avg.** | **Stdev** | **Min** | **Max** |
| Tensile | EMT | extension | % | 59,41 | 2,85 | 56,80 | 62,49 |
| | LT | linearity | - | 0,99 | 0,06 | 0,89 | 1,04 |
| | WT | Tensile energy | $gF{*}cm/cm^2$ | 14,84 | 1,81 | 12,28 | 16,83 |
| Compression | WC | compression energy | $gF\,cm/cm^2$ | 0,44 | 0,29 | 0,10 | 0,79 |
| Surface | SMD | roughness | $\mu$ | 4,25 | 1,69 | 1,24 | 5,24 |

Note that for this table, the averages were obtained by testing 10 articles of the same type. From the table, we note the following:

- An extension (EMT) measured according to Kawabata which is higher than 40%, and on average even higher than 55%.
- A material strength remains equal during extension. This means that the force needed to extend the laminate remains stable during extension. This is measured by the tensile linearity according to Kawabata (LT), which is higher than 0.9 and preferably about 1.0, i.e. LT > 0,9 and preferably LT about 1.0.
- A tensile energy (WT) according to Kawabata whereby WT $\geq$ 12 gF/cm.
- A reduced roughness (SMD) and reduced compression energy (WC), which lead to increased softness.
- Smaller standard deviations for at least the tensile and compression properties than the market standard product, which indicates a better reproducibility of these properties.

which result in a unique elastic behavior of the laminate resulting in an improved fit and wearing comfort, together with the selected soft nonwovens.

### 2.2. Compression properties

A compression energy (WC) measured according to Kawabata, that quantifies the energy needed to compress the material to a certain extent. In a preferred embodiment, WC ≤ 0,65 gF/cm.

A fabric is considered softer than another when it needs less energy to being compressed. The ability to recover to its original shape is measured by the compressional resilience (RC).

### 2.3. Surface properties

The surface friction or COF (MIU) is significantly correlated with the perception of hardness, opposite of softness. A higher MIU indicates a material that has a high degree of friction and resists drag.

The geometrical roughness (SMD) corresponds to the roughness in terms of geometrical shape of the material. A lower SMD indicates a flatter material with less geometrical shape.

Both lower MIU and SMD are positive for the softness. The laminate may comprise a soft 'wave' pattern, created preferably in combination with the soft nonwovens, resulting in an unique bulk softness, quantified according to Kawabata by the roughness (SMD). In a preferred embodiment SMD ≤ 5 μ.

### 3. **Softness evaluation**

### 3.1. Softness of the elastic laminate

The laminate is a three layer structure with two soft nonwovens and an elastic film, laminated to each other, preferably in a discontinuous bonding region, and preferably by ultrasonic bonding. The elastic film is put under a specific tension and gives flexibility properties to the laminate. The nonwovens both have a very smooth surface. Due to the lamination process, folds are created in the laminate which increases bulk and changes the sheet topology. These factors decrease the 'stiffness' and increase flexibility.

- Tensile properties: EMT and/or WT - strength and elasticity
- Compression properties: WC - bulk effect
- Surface properties: geometrical, smooth surface

### 4. **Formability and softness of the described absorbent article**

The laminate consists out of a three layer structure with two soft nonwovens and an elastic film. The elastic film is activated by elongation and laminated to the nonwovens, in a discontinuous bonding region, and preferably by ultrasonic bonding. The nonwovens both have a very smooth surface. Due to the lamination process, folds are created in the laminate which increases bulk and changes the sheet topology. These factors decrease the stiffness and increases the flexibility. The combination of the lamination technology and the soft raw materials result in a very flexible, soft and bulky laminate. This gives an unique adult incontinence pull up with a unique softness and wearing comfort which can be quantified according to Kawabata.

Formability (Fit & comfort) according to Kawabata is obtained from the formula:

$$Formability = F = WT + \sqrt{EMT}$$

Softness according to Kawabata is obtained from the formula:

$$Softness = EMT / SMD + 1/ WC$$

|  | **Ref. 1** | **Ref. 2** | **Ref. 3** |  | **Ref. 4** | **Ref. 4** | **Average Ref. 4** |
|---|---|---|---|---|---|---|---|
| Formability | 6,3 | 15,3 | 22,6 |  | 23,6 | 24,5 | **24,0** |
| Softness | 12,3 | 10,3 | 31,9 |  | 22,8 | 16,5 | **18,9** |

Ref.1: Cotton-knitted goods
Ref.2: Market Standard Protective Underwear pull-up material
Ref.3: Standard laminates of nonwovens with lycra elastics
Ref.4: elastic laminates according to the present invention.

## 5. Emtec - TSA - Tissue softness Analyser

Based on a new measuring method, invented in 2004 by Dipl. Phys. Giselher Gruener, which simulates the human feeling of a tissue sample, a measuring device was developed by Emtec Electronic Leipzig. It delivers three basic paramaters of the human hand feeling.

The instruments collects three important factors for human sensation:

- Fiber softness
- Texture roughness
- Stiffness

This device is on the way to become a standard measuring device in the tissue industry and its technology can be applied on the nonwovens and laminates used in the hygienic industry.

**Principle:**

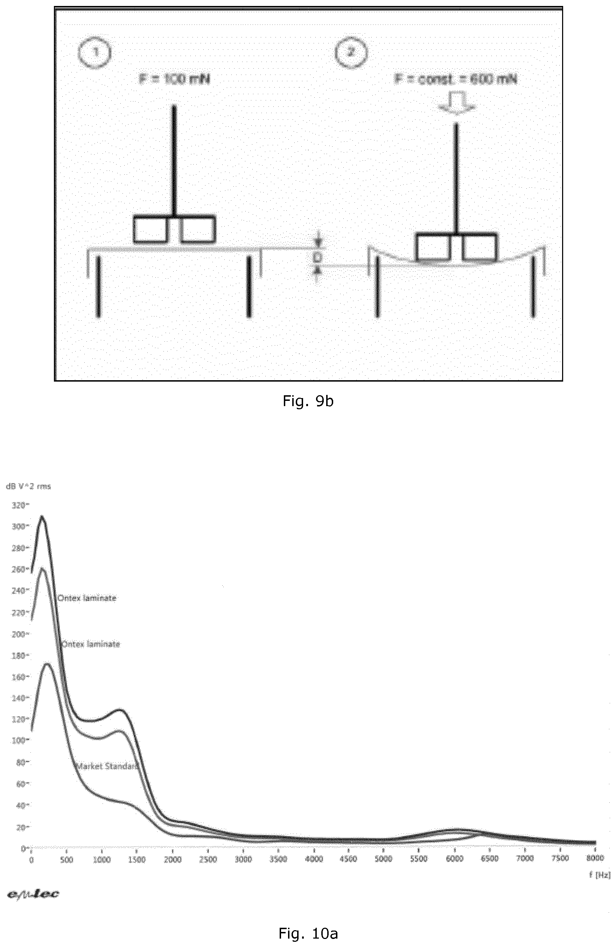

See also FIGS. 9a-b for reference concerning the principle and test conditions for the TSA.

The motion of blades generates, depending on the type of tissue, different types of vibrations or sounds, which are detected by a vibration sensor. Afterwards that test piece will be deformed measuring the elastic properties.

Vertical vibration of the sample is caused by motion of the blade over the surface of the sample. The amplitude of the vibrations depends on the height of the structure (creping, embossing,...). This results in the TS750 peak - texture peak, which is the first peak of the resulting spectrum. The higher the peak, the rougher the texture.

The horizontal vibration of the blade itself is caused by the fibers itself when moving of the surface. This results in the TS7 peak - softness peak. The resonance frequency of the blades itself is at approx. at 6500 Hz. The higher the peak, the lower the softness is of the sample.

In the second step, the deformation of the sample is measured, which correlates with the stiffness of the product. The blade applies a defined load in three cycles in the vertical direction onto the sample. The higher the value of deformation, the higher the flexibility of the product.

**Test conditions:**

Instrument: Emtec - TSA - tissue softness analyser

All measurements must be performed under standard climatic conditions ISO CEN DIN 20187: (23°C ($\pm$ 0,5°C) and 50% ($\pm$ 2%) rel. humidity).

Testpiece: surface of 100 cm$^2$ (diameter 112.8 mm).

Capiler: 100 um

Measing force: 100 mN, measuring duration approx. 45sec.

Density: 140 g/m$^2$

Rotation speed: 2 rps [1/s]

Blade temperature: 23°C $\pm$ 0,5°C

Number of plies: 3

E/D/P/H measurement at 10/600 mN

**Results**

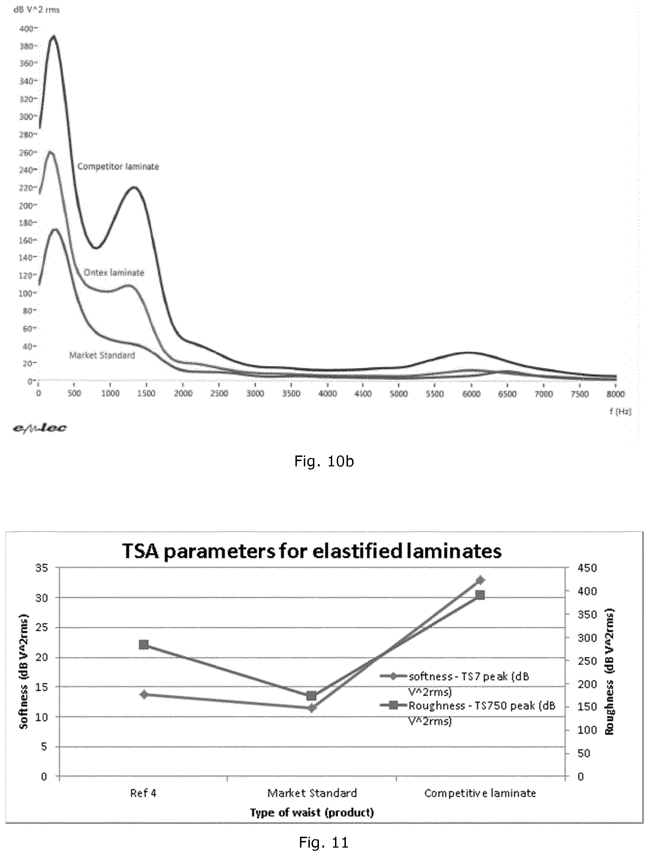

See **FIGS. 10a-b and 11** which illustrate the results of the TSA on samples of the laminate according to the present invention and comparison with Market Standard and competitive laminate.

| Laminate | softness - TS7 peak (dB V^2rms) | Roughness - TS750 peak (dB V^2rms) | Stiffness - D (N/mm) |
|---|---|---|---|
| Ref.4 laminate | 13,80 | 284 | 4,11 |
| Market Standard | 11,4 | 172 | 4,05 |
| Competitive laminate | 32,9 | 391 | 2,85 |

The present laminate shows a very low softness peak (TS7 peak) in combination with a high texture peak (TS750 peak). This results in the unique bulky softness of the laminate.

Therefore, in a preferred embodiment, the laminate comprises a TS750 peak of at least 200 dB $V^2_{rms}$ if the laminate comprises a TS7 peak of at most 25 dB $V^2_{rms}$, more preferably is the laminate comprises a TS7 peak of at most 20 dB $V^2_{rms}$, i.e.

If the TS7 peak $\leq$ 25 dB V^2rms, the TS750 peak $\geq$ 200 dB V^2rms

If the TS7 peak $\leq$ 20 dB V^2rms, the TS750 peak $\geq$ 200 dB V^2rms

## Claims

1. An elastic laminate for absorbent articles comprising: a first nonwoven web, a second nonwoven web and an elastic film laminated between the first and the second nonwoven webs, wherein said elastic laminate comprises a softness according to Kawabata which is higher than 13 and/or a TS750 peak of at least 200 dB $V^2_{rms}$ if the laminate comprises a TS7 peak of at most 25 dB $V^2_{rms}$, as measured according to the tissue softness analyser, whereby said first nonwoven web and/or preferably the second nonwoven web are laminated to the elastic film in a discontinuous bonding region, wherein said elastic film is stretched during lamination, wherein said elastic film is activated by elongation and laminated to the nonwovens and whereby said laminate comprises less than 1 g/m$^2$ of adhesive, whereby said first and/or preferably said second nonwoven comprise:

   - an elongation in a machine direction of at least 60%;
   - a stiffness in a machine direction of at most 35 mN;
   - a stiffness in a cross direction of at most 20 mN, and/or
   - a kinetic coefficient of friction (COF kin) of at most 0,30.

2. The elastic laminate of claim 1 wherein wherein said elastic laminate comprises an extensibility according to Kawabata which is higher than 40 %.

3. An elastic laminate according to claims 1 or 2, whereby said elastic film is stretched during lamination by at least 300%.

4. An elastic laminate according to any of the previous claims comprising a formability according to Kawabata which is higher than 20.

5. An absorbent article comprising an elastic laminate according to any of the previous claims.

6. An absorbent article according to claim 5, comprising a nonwoven front sheet and a nonwoven back sheet, and an elastic film laminated therebetween, thereby forming said elastic laminate, more preferably whereby said laminate is at least partly disposed in a front section and/or a back section of said absorbent article.

7. Use of an elastic laminate according to any claims 1 to 4 for manufacturing an absorbent article.

8. Process for manufacturing an elastic laminate according to any of claims 1 to 4, comprising the steps of: providing an elastic film and laminating, preferably mechanically bonding, the elastic film between a first nonwoven web and a second nonwoven web in a discontinuous bonding region using less than 1 g/m$^2$ of adhesive, whereby said elastic film is stretched during lamination, wherein said elastic film is activated by elongation and laminated to the nonwovens, thereby obtaining an elastic laminate comprising a softness according to Kawabata which is higher than 13 and/or a TS750 peak of at least 200 dB $V^2_{rms}$ if the laminate comprises a TS7 peak of at most 25 dB $V^2_{rms}$, as measured according to the tissue softness analyser, whereby said first and/or preferably said second nonwoven comprise

   - an elongation in a machine direction of at least 60%;
   - a stiffness in a machine direction of at most 35 mN;
   - a stiffness in a cross direction of at most 20 mN, and/or
   - a kinetic coefficient of friction (COF kin) of at most 0,30.

9. Process according to claim 8, whereby said elastic laminate comprises an extensibility according to Kawabata which is higher than 40 %.

**10.** Process according to claim 8 or 9, wherein said elastic film is extended during lamination by at least 300%.

**Patentansprüche**

**1.** Elastisches Laminat für absorbierende Artikel, umfassend: eine erste Vliesstoffbahn, eine zweite Vliesstoffbahn und eine elastische Folie, die zwischen der ersten und der zweiten Vliesstoffbahn laminiert ist, wobei das elastische Laminat eine Weichheit gemäß Kawabata, die höher als 13 ist, und/oder einen TS750-Peak von mindestens 200 dB $V^2_{rms}$ umfasst, falls das Laminat einen TS7-Peak von höchstens 25 dB $V^2_{rms}$ umfasst, wie gemäß dem Gewebeweichheitsanalysator gemessen, wobei die erste Vliesstoffbahn und/oder vorzugsweise die zweite Vliesstoffbahn mit der elastischen Folie in einem diskontinuierlichen Verbindungsbereich laminiert sind, wobei die elastische Folie während einer Laminierung auseinandergezogen wird, wobei die elastische Folie durch Streckung aktiviert und auf die Vliesstoffe laminiert wird, und wobei das Laminat weniger als 1 g/m$^2$ Klebemittel umfasst, wobei der erste und/oder vorzugsweise der zweite Vliesstoff umfassen:

- eine Streckung in einer Maschinenrichtung von mindestens 60 %;
- eine Steifigkeit in einer Maschinenrichtung von höchstens 35 mN;
- eine Steifigkeit in einer Querrichtung von höchstens 20 mN und/oder
- einen kinetischen Reibungskoeffizienten (COF kin) von höchstens 0,30.

**2.** Elastisches Laminat nach Anspruch 1, wobei das elastische Laminat eine Dehnbarkeit gemäß Kawabata umfasst, die höher als 40 % ist.

**3.** Elastisches Laminat nach Anspruch 1 oder 2, wobei die elastische Folie während der Laminierung um mindestens 300 % auseinandergezogen wird.

**4.** Elastisches Laminat nach einem der vorstehenden Ansprüche, umfassend eine Formbarkeit gemäß Kawabata, die höher als 20 ist.

**5.** Absorbierender Artikel, umfassend ein elastisches Laminat nach einem der vorstehenden Ansprüche.

**6.** Absorbierender Artikel nach Anspruch 5, umfassend eine vordere Vliesstofflage und eine hintere Vliesstofflage und eine elastische Folie, die dazwischen laminiert ist, wobei dadurch das elastische Laminat geformt wird, wobei das Laminat mehr bevorzugt mindestens teilweise in einem vorderen Abschnitt und/oder einem hinteren Abschnitt des absorbierenden Artikels angeordnet ist.

**7.** Verwendung eines elastischen Laminats nach einem der Ansprüche 1 bis 4 zum Herstellen eines absorbierenden Artikels.

**8.** Verfahren zum Herstellen eines elastischen Laminats nach einem der Ansprüche 1 bis 4, umfassend die Schritte: Bereitstellen einer elastischen Folie und Laminieren, vorzugsweise mechanisches Verbinden, der elastischen Folie zwischen einer ersten Vliesstoffbahn und einer zweiten Vliesstoffbahn in einem diskontinuierlichen Verbindungsbereich unter Verwendung von weniger als 1 g/m$^2$ Klebemittel, wobei die elastische Folie während der Laminierung auseinandergezogen wird, wobei die elastische Folie durch Streckung aktiviert und auf die Vliesstoffe laminiert wird, wobei dadurch ein elastisches Laminat erhalten wird, umfassend eine Weichheit gemäß Kawabata, die höher als 13 ist, und/oder einen TS750-Peak von mindestens 200 dB $V^2_{rms}$, falls das Laminat einen TS7-Peak von höchstens 25 dB $V^2_{rms}$ umfasst, wie gemäß dem Gewebeweichheitsanalysator gemessen, wobei der erste und/oder vorzugsweise der zweite Vliesstoff umfassen

- eine Streckung in einer Maschinenrichtung von mindestens 60 %;
- eine Steifigkeit in einer Maschinenrichtung von höchstens 35 mN;
- eine Steifigkeit in einer Querrichtung von höchstens 20 mN und/oder
- einen kinetischen Reibungskoeffizienten (COF kin) von höchstens 0,30.

**9.** Verfahren nach Anspruch 8, wobei das elastische Laminat eine Dehnbarkeit gemäß Kawabata umfasst, die höher als 40 % ist.

**10.** Verfahren nach Anspruch 8 oder 9, wobei die elastische Folie während der Laminierung um mindestens 300 % gedehnt wird.

**Revendications**

1.  Stratifié élastique pour articles absorbants comprenant : une première bande non tissée, une seconde bande non tissée et un film élastique stratifié entre les première et seconde bandes non tissées, dans lequel ledit stratifié élastique comprend une douceur selon Kawabata qui est supérieure à 13 et/ou un pic TS750 d'au moins 200 dB $V^2_{rms}$ si le stratifié comprend un pic TS7 d'au plus 25 dB $V^2_{rms}$, telle que mesurée selon l'analyseur de souplesse de tissu, selon lequel ladite première bande non tissée et/ou de préférence la seconde bande non tissée sont stratifiées au film élastique dans une région de liaison discontinue, dans lequel ledit film élastique est étiré pendant le stratification, dans lequel ledit film élastique est activé par allongement et stratifié sur les non-tissés et moyennant quoi ledit stratifié comprend moins de 1 g/m² d'adhésif,
    selon lequel ledit premier et/ou de préférence ledit second non-tissé comprennent :

    - un allongement dans un sens machine d'au moins 60 % ;
    - une rigidité dans un sens machine d'au plus 35 mN ;
    - une rigidité dans une direction transversale d'au plus 20 mN, et/ou
    - un coefficient de frottement cinétique (COF kin) d'au plus 0,30.

2.  Stratifié élastique selon la revendication 1, dans lequel ledit stratifié élastique comprend une extensibilité selon Kawabata qui est supérieure à 40 %.

3.  Stratifié élastique selon les revendications 1 ou 2, selon lequel ledit film élastique est étiré pendant la stratification d'au moins 300 %.

4.  Stratifié élastique selon l'une quelconque des revendications précédentes, comprenant une aptitude à la formation selon Kawabata qui est supérieure à 20.

5.  Article absorbant comprenant un stratifié élastique selon l'une quelconque des revendications précédentes.

6.  Article absorbant selon la revendication 5, comprenant une feuille avant non tissée et une feuille arrière non tissée, et un film élastique stratifié entre elles, formant ainsi ledit stratifié élastique, plus préférablement selon lequel ledit stratifié est au moins partiellement disposé dans une section avant et/ou une section arrière dudit article absorbant.

7.  Utilisation d'un stratifié élastique selon l'une quelconque des revendications 1 à 4 pour la fabrication d'un article absorbant.

8.  Procédé de fabrication d'un stratifié élastique selon l'une quelconque des revendications 1 à 4, comprenant les étapes consistant à : fournir un film élastique et stratifier, de préférence lier mécaniquement, le film élastique entre une première bande non tissée et une seconde bande non tissée dans une région de liaison discontinue à l'aide de moins de 1 g/m² d'adhésif, selon lequel ledit film élastique est étiré pendant la stratification, dans lequel ledit film élastique est activé par allongement et stratifié aux non-tissés, permettant ainsi d'obtenir un stratifié élastique comprenant une douceur selon Kawabata qui est supérieure à 13 et/ou un pic TS750 d'au moins 200 dB $V^2_{rms}$ si le stratifié comprend un pic TS7 d'au plus 25 dB $V^2_{rms}$, telles que mesurées selon l'analyseur de douceur de tissu, selon lequel ledit premier et/ou de préférence ledit second non-tissé comprennent

    - un allongement dans un sens machine d'au moins 60 % ;
    - une rigidité dans un sens machine d'au plus 35 mN ;
    - une rigidité dans une direction transversale d'au plus 20 mN, et/ou
    - un coefficient de frottement cinétique (COF kin) d'au plus 0,30.

9.  Procédé selon la revendication 8, selon lequel ledit stratifié élastique comprend une extensibilité selon Kawabata supérieure à 40 %.

10. Procédé selon la revendication 8 ou 9, dans lequel ledit film élastique est étendu pendant le stratification d'au moins 300 %.

Fig. 1

Fig. 2

Fig. 3

Fig. 4

Fig. 5

Fig. 6

Fig. 7a

Fig. 7b

Fig. 7c

Fig. 8

Fig. 9a

Fig. 9b

Fig. 10a

Fig. 10b

Fig. 11

**EP 2 891 480 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8052665 B2
- CA 2649926
- US 2009208703 A
- WO 2013002691 A
- WO 2009133508 A

**Non-patent literature cited in the description**

- **KAWABATA, S.** ; **NIWA, M** ; **WANG, F.** Objective Hand Measurement of Non Woven Fabrics. *Textile Research J*, 1994, 597
- **BISHOP, D. P.** Fabrics: Sensory and Mechanical Properties. *Textile Progress*, 1995, vol. 26 (3)
- **MATSUO, T.** ; **NASU, N.** ; **SAITO, M.** Study on the Hand. Part II. The Method of Measuring Hand. *J. Text. Mach. Soc. Jap*, 1971, vol. 17.3, 92
- **SUEO KAWABATA**. The Standardization and Analysis of Hand Evaluation. July 1980